# EUROPÄISCHE PATENTANMELDUNG

(11) **EP 2 130 832 A1**
(43) Veröffentlichungstag der Anmeldung: **09.12.2009**
(21) Anmeldenummer: 08151840.9
(22) Anmeldetag: 22.02.2008
(51) Int. Cl.: C07F 1/00, A61K 31/00, A61P 35/00

(54) **Kupfer-Organokomplexe, deren Verwendung als Antitumormittel und zum Schutz gesunden Gewebes vor ionisierender Strahlung**

(71) Anmelder: Tatarsky, Valeriy, Prof. Dr., 14469 Potsdam (DE); Charité - Universitätsmedizin Berlin, 10117 Berlin (DE)
(72) Erfinder: Die Erfindernennung liegt noch nicht vor
(74) Vertreter: Ziebig, Marlene

(57) **Zusammenfassung**

Die vorliegende Erfindung betrifft Kupfer-Organokomplexe und sie enthaltende pharmazeutische Zusammensetzungen. Sie sind insbesondere zur Behandlung von Krankheiten, die durch hyperproliferative Zellen verursacht werden, verwendbar und schützen daneben gesundes Gewebe vor ionisierender Strahlung. Sie werden durch Umsetzung eines Kupfer(II)acylats bei saurem bis neutralem pH-Wert in einem Chlorofom/Methanolgemisch mit einer organischen Verbindung ausgewählt aus 4-[Bis(2-chlorethyl)-amino]-D,L-phenylalanin (Sarcolysin), dessen Hydrochlorid, N-(2-Furanidil)-5-fluoruracil (Tegafur) und Aminocarbonylaziridin (2-Carbamoylaziridin-Leacadin) hergestellt.

## Beschreibung

Die vorliegende Erfindung betrifft Kupfer-Organokomplexe und sie enthaltende pharmazeutische Zusammensetzungen. Sie sind insbesondere zur Behandlung von Krankheiten, die durch hyperproliferative Zellen verursacht werden, verwendbar und schützen daneben gesundes Gewebe vor ionisierender Strahlung. Sie werden durch Umsetzung eines Kupfer(II)acylats bei saurem bis neutralem pH-Wert in einem Chloroform/Methanolgemisch mit einer organischen Verbindung ausgewählt aus 4-[Bis(2-chlorethyl)-amino]-D,L-phenylalanin (Sarcolysin), dessen Hydrochlorid, N-(2-Furanidil)-5-fluoruracil (Tegafur) und Aminocarbonylaziridin (2-Carbamoylaziridin - Leacadin) hergestellt.

Hyperproliferative Zellen sind die Ursache für verschiedene Krankheiten, insbesondere die unter dem Begriff Krebs zusammengefassten Krankheitsbilder mit oft tödlichem Ausgang. Durch eine übermäßige Proliferation entsteht ein Ungleichgewicht zwischen Gewebeneubildung und dem gesteuerten Absterben von Zellen aus dem Gewebeverband. Die natürliche Homöostase wird gestört. Diese sensible Balance zwischen Gewebeauf- und abbau wird durch den Prozess der Apoptose reguliert. Die Apoptose bezeichnet den programmierten Zelltod, den jede Zelle ausführen kann. Auf bestimmte Signale hin werden intrazelluläre Prozesse ausgelöst, die dazu führen, dass sich die Zelle selbst zerlegt, ohne dabei Entzündungsprozesse hervorzurufen. Auf diese Weise wird die übermäßige Proliferation von Zellen und Geweben verhindert.

Bei der Behandlung von Erkrankungen, die durch übermäßig proliferierende Zellen verursacht werden, versucht man in der klinischen Praxis neben der chirurgischen Entfernung lokalisierbarer Tumoren die Tumorzellen zu töten und das Gleichgewicht durch zytotoxische Maßnahmen wie Chemotherapie, Strahlentherapie und Hyperthermie wiederherzustellen. Dabei zeigt sich jedoch immer wieder, dass ein Teil der malignen Tumore sehr früh eine Strahlen- oder Chemoresistenz entwickelt oder gar primär therapierefraktär ist. Teilweise unterscheiden sich Primärtumor und Metastasen auch sehr in ihrem Ansprechverhalten auf die jeweilige Therapie. Durch diese Resistenzen bleiben viele Tumortherapien noch unbefriedigend. Besonders trifft das auf die Behandlung des Rezidivs der akuten lymphoblastischen Leukämie (ALL) im Kindesalter zu. Trotz aggressiver zytotoxischer Therapie versterben etwa 60% der an einem ALL-Rezidiv erkrankten Kinder. Weitere schwer therapierbare Tumorerkrankungen sind beispielsweise das Mamma-, Bronchial-, Schilddrüsen- und Prostatakarzinom wie auch Melanom, Neuroblastom, Medulloblastom, Astrozytom und Glioblastom. Aber auch gutartige Erkrankungen, die auf hyperprolieferative Zellen zurückgehen, werden mit zytostatischen Medikamenten behandelt, die in ihrer therapeutischen Potenz noch erheblich verbesserungswürdig sind.

In WO 03/004014 A1 sind bereits monokristalline Diketon-Kupferkomplexe von Melphalan , Tegafur und Leacadin beschrieben, die als Antitumormittel Verwendung finden können. Melphalan, Tegafur und Leacadin sind auch selbst als Zytostatika bekannt.

Es gibt jedoch ein starkes Interesse und großes Bedürfnis an der Entwicklung weiterer Substanzen, die Heilungserfolge und Überlebenschancen verbessern können. Der Erfindung lag deshalb die Aufgabe zugrunde, Verbindungen zu finden, die hochselektiv gegen übermäßig proliferierende, arzneimittelresistente Zellen wirken und die zur Behandlung von Tumorerkrankungen und Leukämien geeignet sind, ohne gesunde Zellen übermäßig zu schädigen. Es war eine weitere Aufgabe der Erfindung, Verbindungen bereitzustellen, die bei der medizinischen Anwendung von ionisierender Strahlung auf den Menschen, um Krankheiten zu heilen oder deren Fortschreiten zu verzögern, gesundes Gewebe schützen.

Die Aufgabe der Erfindung wird gelöst durch neue Kupfer-Organokomplexe, die durch Umsetzung eines Kupfer(II)acylats bei saurem bis neutralem pH-Wert in einem Chlorofom/Methanolgemisch mit einer organischen Verbindung erhalten werden können. Erfindungsgemäß ist die organische Verbindung ausgewählt aus 4-[Bis(2-chlorethyl)-amino]-D,L-phenylalanin (D,L-Melphalan, Sacrolysin), dessen Hydrochlorid, N-(2-Furanidil)-5-fluoruracil (Tegafur) und Aminocarbonylaziridin (2-Carbamoylaziridin-Leacadin). Bevorzugt werden Kupfer(II)acetat oder Kupfer(II) propionat als Kupferacylat eingesetzt.

Es wurde nun gefunden, dass die erfindungsgemäßen Kupfer-Organokomplexe hochselektiv gegen übermäßig proliferierende, arzneimittelresistente Zellen sind und zur Behandlung von Tumorerkrankungen und Leukämien eingesetzt werden können. Darüber hinaus sind sie überraschend in der Lage, gesundes Gewebe vor Strahlenschäden, z. B. bei der Strahlenbehandlung nach Tumorresektion oder bei der Tumorbehandlung mittels Bestrahlung, zu schützen, so dass sie in der Strahlentherapie Verwendung finden können. Weiterhin wurde gefunden, dass die erfindungsgemäßen Kupfer-Organokomplexe in Kombination mit an sich bekannten Zytostatika bei der Behandlung von Tumorerkrankungen eine synergistische Wirkung zeigen. Darüber hinaus zeigen die erfindungsgemäßen Kupfer-Organokomplexe eine sehr ausgeprägte antioxidative Wirkung und sind damit als Antioxidationsmittel auch zur Behandlung inflammatorischer Erkrankungen, die z. B. auch mit Tumorerkrankungen einhergehen können, einsetzbar.

Die Herstellung der erfindungsgemäßen Kupferkomplexe erfolgt bevorzugt durch Vermischen und Erhitzen von Lösungen der Ausgangssubstanzen. Beim Abkühlen fällt der Komplex aus und wird durch mehrfaches Waschen mit einem Chloroform/Methanolgemisch als Lösungsmittel gereinigt und dann getrocknet. Für den Erfolg der Umsetzung ist die Einhaltung eines engen pH-Bereichs im sauren bis neutralen Milieu erforderlich. Der Komplex ist eine mikrokristalline Verbindung, in der das Kupfer tetragonal vorliegt und die organische Verbindung über Sauerstoff - und/oder Stickstoffatome an das Kupfer gebunden ist.

Erfindungsgemäß werden vorzugsweise folgende Kupfer-Organokomplexe bereitgestellt:
a) Kupfer-Sarcolysin-Hydrochlorid-Komplex (Komplex A), der einen Schmelzpunkt von 147°C aufweist sowie die folgende elementaranalytische Zusammensetzung besitzt: Cu: 15%, C: 37,3%, Cl: 24,4%, N: 6,74% und O: 10,3%, hergestellt z. B. durch Umsetzung von Sarcolysin-Hydrochlorid und Kupfer(II)acetat bei einem pH von ca. 2 bis ca. 3.
b) Kupfer-Sarcolysin-Komplex (Komplex B), der einen Schmelzpunkt von 177°C aufweist sowie die folgende elementaranalytische Zusammensetzung besitzt: Cu: 9,2%, C: 45,3%, Cl: 20,6%, N: 8,1% und O: 11,6%, hergestellt z. B. durch Umsetzung von Sarcolysin und Kupfer(II)acetat bei einem pH von ca. 6 bis ca. 7.
c) Kupfer-Tegafur-Komplex (Komplex C), der einen Schmelzpunkt von 127°C aufweist, hergestellt z. B. durch Umsetzung von Tegafur und Kupfer(II)acetat bei einem pH-Wert von ca. 1.

Gegenstand der Erfindung ist auch eine pharmazeutische Zusammensetzung, welche mindestens einen neuen Kupfer-Organokomplex umfasst sowie gegebenenfalls pharmazeutische Hilfs- und/oder Trägerstoffe.

Die pharmazeutische Zusammensetzung wird nach an sich bekannten Verfahren hergestellt, wobei die erfindungsgemäße Komplexverbindung vorzugsweise in Kombination mit geeigneten pharmazeutischen Trägerstoffen bereitgestellt wird. Dabei beträgt der Wirkstoffgehalt dieser Zusammensetzung üblicherweise 0,1 bis 99,5 Gew.-%, vorzugsweise 0,5 bis 95 Gew.-%, der Gesamtmischung.

Die erfindungsgemäße pharmazeutische Zusammensetzung kann in unterschiedlichen Applikationsformen bereitgestellt werden. Sie besteht in der Regel aus mindestens einer erfindungsgemäßen Komplexverbindung und nichttoxischen, pharmazeutisch verträglichen Arzneimittelträgern, die als Zumischung oder Verdünnungsmittel, beispielsweise in fester, halbfester oder flüssiger Form oder als Umhüllungsmittel beispielsweise in Form einer Kapsel, eines Tablettenüberzugs, eines Beutels oder eines anderen Behältnisses für den therapeutisch aktiven Bestandteil zur Anwendung kommen. Ein Trägerstoff kann z. B. als Vermittler für die Arzneimittelaufnahme in den Körper, als Formulierungshilfsmittel, als Süßungsmittel, als Geschmackskorrigens, als Farbstoff oder als Konservierungsmittel dienen.

Die erfindungsgemäße pharmazeutische Zusammensetzung wird vor allem peroral, intraperitoneal, intratracheal, intraabdominal, intravenös, transdermal oder intramuskulär verabreicht oder in einer Verabreichungsform mittels Mikroklistier. Auch eine pulmonale Applikation ist möglich.

Zur parenteralen Anwendung der pharmazeutischen Zusammensetzung dienen steril injizierbare wässrige Lösungen, isotonische Salzlösungen oder sonstige Lösungen. Wässrige Suspensionen können Suspendiermittel, z. B. Natriumcarboxymethylcellulose, Methylcellulose, Hydroxypropylcellulose, Natriumalginat, Polyvinylpyrrolidon, Tragant oder Gummi arabicum; Dispergier- und Benetzungsmittel, z. B. Polyoxyethylenstarat, Heptadecaethyleoxycatanol, Polyoxyethylensorbitolmonooleat oder Lecithin; Konservierungsmittel, z. B. Methyl- oder Propylhydroxybenzoat; Geschmacksmittel, Süßungsmittel, z. B. Saccharose, Natriumcyclamat, Glucose, Invertzuckersirup, enthalten.

Ölige Suspensionen können z. B. Erdnuss-, Oliven-, Sesam-, Kokos- oder Paraffinöl und Verdickungsmittel, wie z. B. Bienenwachs, Hartparaffin oder Cetylalkohol, enthalten; ferner Süßungsmittel, Geschmacksmittel und Antioxidantien.

In Wasser dispergierbare Pulver und Granulate können die erfindungsgemäße Komplexverbindung in Mischung mit Dispergier-, Benetzungs- und Suspendiermitteln, z. B. den oben erwähnten ggf. mit SüßungsmitteIn, GeschmacksmitteIn und Farbstoffen enthalten.

Emulsionen können z. B. Erdnuss-, Oliven-, oder Paraffinöl neben Emulgatoren wie z. B. Tragant, Gummi arabicum, Polyoxyethylensorbitanmonooleat; und Geschmacks- und Süßungsmittel enthalten.

Wässrige Lösungen können Konservierungsmittel, z. B. Methyl- oder Propylhydroxybenzoat; Verdickungsmittel, Geschmacksmittel, Süßungsmittel, z. B. Saccharose, Natriumcyclamat, Glucose, Invertzuckersirup sowie Farbstoffe enthalten.

Bevorzugt werden als Verdünnungs- und Lösungsmittel Olivenöl, DMSO, Tween 60 oder 80 und physiologische Kochsalzlösung verwendet.

Zur peroralen Anwendung können z. B. Tabletten, überzogene Tabletten, KapseIn, z. B. aus Gelatine, dispergierbare Pulver, Granulate, wässrige und ölige Suspensionen, Emulsionen, Lösungen oder Sirupe kommen. Tabletten können inerte Füllmittel, z.B. Stärken, Lactose, mikrokristalline Cellulose, Glucose, Calciumcarbonat oder Natriumchlorid; Bindemittel, z. B. Stärken, PEGe, PVP, Gelatine Cellulosederivate, Alginate oder Gummi arabicum; Gleitmittel, z. B. Magnesiumstearat, Glycerinmonostearat, Stearinsäure, Silikonöle oder Talkum, Zerfallsmittel, Geschmackskorrigentien oder Farbstoffe enthalten.

Vorzugsweise wird die pharmazeutische Zusammensetzung in Form einer feinen Dispersion in Öl oder als intravenöse Injektions- oder Infusionslösung oder in Tablettenform zur oralen Applikation angewandt. Überraschend entfalten die Kupfer-Organokomplexe ihre Wirkung auch bei oraler Applikation in vollem Umfang, was erhebliche Vorteile in der Handhabung und Erleichterungen für den Patienten bedeutet.

Vorteilhafterweise liegt die pharmazeutische Zubereitung des Wirkstoffs in Form von Einheitsdosen vor, die auf die gewünschte Verabreichung abgestimmt sind. Eine Einheitsdosis kann z. B. eine Tablette, eine Kapsel, ein Suppositorium oder eine angemessene Volumenmenge eines Pulvers, eines Granulates, einer Lösung, einer Emulsion oder einer Suspension oder Dispersion sein.

Im Allgemeinen werden die erfindungsgemäßen Komplexe in einer Tagesdosis von 0,01 bis 10 mg/kg Körpergewicht, gegebenenfalls in Form mehrerer Einzelgaben zur Erzielung des gewünschten Ergebnisses verabreicht. Eine Einzelgabe enthält den oder die Komplexe in Mengen von 0,01 bis 10 mg/kg Körpergewicht.

Es wurde festgestellt, dass sich die erfindungsgemäßen Kupferkomplexe nahezu ausschließlich im Tumorgewebe akkumulieren und auch in der Lage sind, die Blut-Hirn-Schranke zu überwinden. Die neuen Kupfer-Organokomplexe werden deshalb vorzugsweise zur Behandlung von einer Reihe von Tumorerkrankungen eingesetzt. Die erfindungsgemäßen Verbindungen lassen sich für die Behandlung von sehr vielen und unterschiedlichen Krankheitsbildern einsetzen, insbesondere solchen, die auf hochproliferative Zellen zurückzuführen sind, so z. B. zur Behandlung von malignen Erkrankungen des Knochenmarks und anderer blutbildender Organe, Leukämien, soliden Tumoren, epithelialen Tumoren, Sarkomen und malignen und semimalignen Erkrankungen der Haut. Die erfindungsgemäßen Komplexe führen in starkem Maße die Apoptose von Krebszellen herbei, selbst wenn diese gegen herkömmliche Therapeutika resistent sind. So induzieren sie in malignen Zellen die Expression des Proteins p53 und eine Öffnung der Cyclosporin A-Poren.

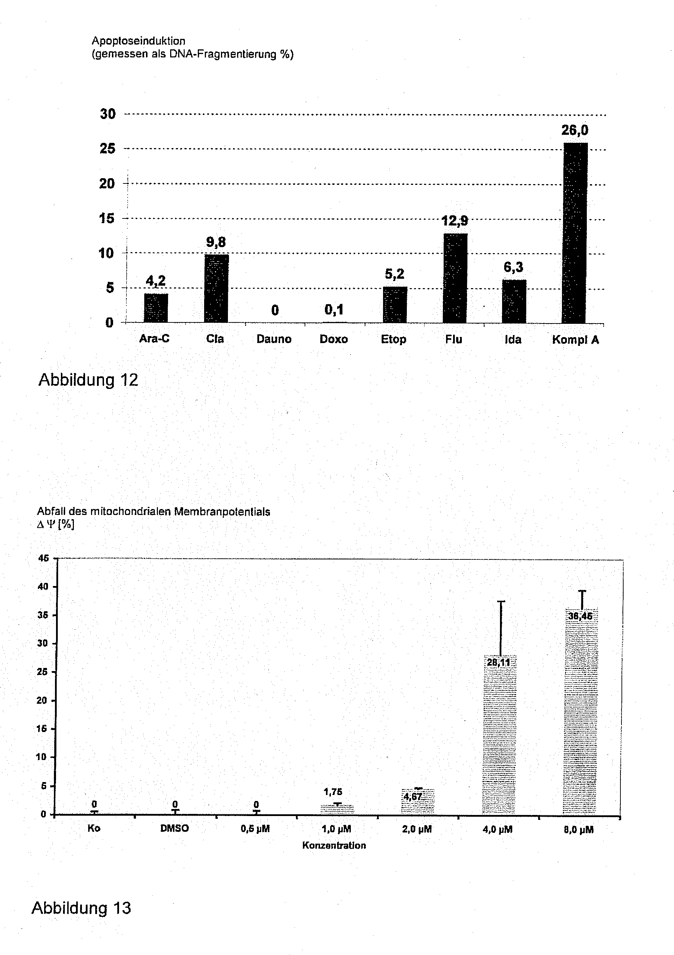

Die erfindungsgemäßen Kupfer-Organokomplexe bewirken z. B. in gegen Daunorubicin und Doxorubicin resistenten Leukämiezellen eine zwei- bis sechsfach erhöhte Apoptoseinduktion im Vergleich zu herkömmlichen Zytostatika (vgl. Beispiel 5 und Abb. 12).

Eine andere bevorzugte Anwendung liegt in der Strahlentherapie, vor allem zum Schutz gesunden Gewebes vor ionisierenden Strahlen. Strahlentherapie (auch Strahlenheilkunde, Radiotherapie, Radioonkologie) ist das medizinische Fachgebiet, das sich mit der medizinischen Anwendung von ionisierender Strahlung auf den Menschen und auf Tiere beschäftigt, um Krankheiten zu heilen oder deren Fortschreiten zu verzögern. Die Strahlentherapie dient der direkten Zerstörung der Krebszellen. Das Gebiet der Strahlentherapie umfasst auch die medikamentösen und physikalischen Verfahren zur Radiosensibilisierung und Verstärkung der Strahlenwirkung am Tumor (Radiochemotherapie), unter Berücksichtigung von Schutzmaßnahmen des gesunden Gewebes. Als ionisierende, hochenergetische Strahlung werden vorwiegend Gammastrahlung, Röntgenbremsstrahlung und Elektronen eingesetzt. In den letzten Jahren wurden auch Anlagen zur Behandlung mit Neutronen, Protonen und schweren Ionen errichtet. Erfindungsgemäß werden die Kupfer-Organokomplexe zum Schutz des gesunden Gewebes, gegebenenfalls in Kombination mit bekannten strahlentherapeutischen Maßnahmen und Mitteln angewendet. Dabei werden die erfindungsgemäßen Komplexe in den oben beschriebenen Applikationsformen und Dosen angewendet.

In einer weiteren Ausführungsform der Erfindung können die Kupfer-Organokomplexe in Kombination mit mindestens einem weiteren Zytostatikum (Tumortherapeutikum) angewendet werden.

Die erfindungsgemäßen Kuper-Organokomplex-Verbindungen sind insbesondere zur Behandlung von folgenden Tumorerkrankungen geeignet, wobei sie allein oder in Kombination mit strahlentherapeutischen Maßnahmen/Mitteln und/oder in Kombination mit herkömmlichen Tumormitteln zum Einsatz kommen: Darmkrebs, Hirntumor, Augentumor, Pankreaskarzinom, Harnblasenkarzinom, Lungenkrebs, Brustkrebs, Ovarialtumor, Halstumor, Hautkrebs, Hodenkrebs, Nierentumor, Keimzelltumor, Leberkrebs, Leukämie, malignes Lymphom, Nerventumor, Neuroblastom, Prostatakrebs, Weichteiltumor, Speiseröhrenkrebs sowie Karzinome bei unbekanntem Primärtumor.

Vorzugsweise wird die pharmazeutische Zusammensetzung einem Patienten mit einer Tumorerkrankung in einer Menge verabreicht, die ausreichend ist, um eine Behandlung des entsprechenden Tumors zu erzielen. Die zu verabreichende Menge der pharmazeutischen Zusammensetzung hängt dabei von mehreren Faktoren ab, wie z. B. der Wahl des spezifischen Kupferkomplexes und gegebenenfalls anderer gleichzeitig verabreichter Pharmazeutika, der Art der Verabreichung (peroral, Infusion, Injektion etc.), der Art und dem Ausmaß der Tumorerkrankung und dem Alter, Gewicht und Allgemeinzustand des Patienten. Die Menge kann ohne weiteres von einem Fachmann auf dem Gebiet der Tumorerkrankung unter Berücksichtigung der oben genannten Faktoren bestimmt werden. Vorzugsweise werden die Kupferkomplexe im Verlauf einer Behandlung in der Regel in Einzeldosen im Bereich von 0,01 mg/kg Körpergewicht des Patienten bis zu 10 mg/kg Körpergewicht des Patienten und besonders bevorzugt von 0,5 bis 5 mg/kg, ganz besonders bevorzugt in Dosierungen von 0,5 bis 1,5 mg/kg Körpergewicht des Patienten verabreicht.

Die Bezeichnung Tumor, wie hierin verwendet, umfasst jede örtliche Zunahme des Gewebevolumens sowie Zellen, bei denen die normale Wachstumsregulation nicht mehr greift und eine ungeregelte Zellteilung stattfindet. Das heißt eine gewebliche Neubildung (z. B. Gewächs, Blastom, Neoplasie) in Form eines spontanen, verschiedenartig enthemmten, autonomen und irreversiblen Überschusswachstums von körpereigenem Gewebe, das in der Regel mit unterschiedlich ausgeprägtem Verlust spezifischer Zell- und Gewebefunktionen verbunden ist.

Die Bezeichnung "Behandlung von Tumoren, wie hierin verwendet, umfasst wenigstens eines der folgenden Merkmale: eine Linderung der mit der Tumorerkrankung verbundenen Symptome, eine Verringerung des Ausmaßes der Tumorerkrankung (z. B. eine Reduktion des Tumorwachstums), eine Stabilisierung des Zustandes der Tumorerkrankung (z. B. eine Inhibierung des Tumorwachstums), eine Verhinderung der weiteren Ausbreitung der Tumorerkrankung (z. B. der Metastasierung), eine Verhinderung des Auftretens oder Wiederauftretens einer Tumorerkrankung, eine Verlangsamung des Verlaufs der Tumorerkrankung (z. B. eine Reduktion des Tumorwachstums) oder eine Verbesserung des Zustandes der Tumorerkrankung (z. B. eine Verkleinerung des Tumors).

Die erfindungsgemäßen Substanzen können durch Herstellungsweise, Elementaranalyse, Schmelzpunkt, UV/VIS-, IR-, EPR- und NMR-Spektren eindeutig charakterisiert werden. Da die erfindungsgemäßen Komplexe sich durch eine Reihe von herausragenden Eigenschaften auszeichnen, sind sie herkömmlichen Chemotherapeutika eindeutig überlegen.

Da sich die erfindungsgemäßen Kupferkomplexe nahezu ausschließlich im Tumorgewebe akkumulieren, sind die Nebenwirkungen einer Behandlung mit diesen erfindungsgemäßen Komplexen vergleichsweise gering. Das eröffnet darüber hinaus auch die Möglichkeit der Verwendung der erfindungsgemäßen Komplexe zum Nachweis von malignen Zellen. So lassen sich die malignen Zellen im Gewebe mit den erfindungsgemäßen Komplexen und üblichen Methoden nachweisen, z. B. mittels Fluoreszenzmikroskopie, wenn die erfindungsgemäßen Komplexe, die mit entsprechenden, dem Fachmann bekannten Fluoreszenzfarbstoffen markiert sind, appliziert werden. Desgleichen können die malignen Zellen im entnommenen Gewebe in vitro mit den erfindungsgemäßen Komplexen nachgewiesen werden.

Die erfindungsgemäßen Komplexe sind in der Lage, die Blut-Hirn-Schranke zu überwinden. Sie können deshalb auch in der Therapie von Hirntumoren eingesetzt werden. Weiterhin bewirkt der Einsatz der erfindungsgemäßen Kupfer-Organokomplexe unter anderem auch eine Verkapselung der Tumore, wodurch deren chirurgische Entfernung erleichtert wird.

Gegenstand der Erfindung sind auch Kombinationspräparate, die die erfindungsgemäßen Kupferkomplexe und an sich bekannte Zytostatika, wie z. B. Cytarabin, Cladribin, Etoposid, Fludarabin oder Idarubicin, umfassen. Dabei besteht ein weiterer Vorteil der erfindungsgemäßen Kupferkomplexe darin, dass sich bei der Anwendung in Kombination mit anderen, herkömmlichen Zytostatika eine erhebliche synergistische Wirkungssteigerung gegenüber der Anwendung der einzelnen Präparate zeigt. Erfindungsgemäß werden also die Kupfer-Organokomplexe in Kombination mit herkömmlichen Zytostatika eingesetzt. Die Applikation der beiden Komponenten des Kombinationspräparates kann gleichzeitig oder sequentiell erfolgen Die herkömmlichen Zytostatika sind bevorzugt ausgewählt aus alkylierenden und quervernetzenden Verbindungen, wie z. B. Stickstofflost-Derivaten, N-Nitroso-Verbindungen, Ethylenimin-(Aziridin-) Derivaten, Platinkomplexen; cytostatischen Antibiotika, wie z. B. Anthracyclinen, Bleomycin und Mitomycin; Antimetaboliten, wie z. B. Folsäure-Antagonisten, Pyrimidin- und Purin-Analoga; Mitosehemmstoffen, wie z. B. Vinca-Alkaloiden und Taxanen; Hormonen und Hormon-Antagonisten .

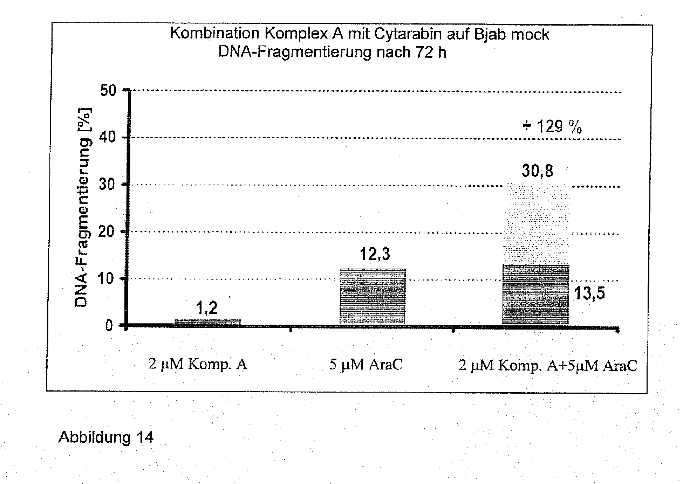

So hat sich z. B. gezeigt, dass die Kombination von Kupfer-Sarcolysin-Hydrochlorid (Komplex A) und einem Antimetaboliten, nämlich Cytarabin (AraC) zu einer Wirkungssteigerung um 129 % führt. Bemerkenswert ist dabei, dass dieser synergistische Effekt bereits mit einer subtherapeutischen Dosis des erfindungsgemäßen Komplexes A eintritt (vgl. Beispiel 7 und Abb. 14).

Das erfindungsgemäße Kombinationspräparat ist somit hervorragend zur Behandlung von Tumorerkrankungen geeignet. Daneben kann es durch die Anwesenheit der erfindungsgemäßen Komplexe im Präparat zum Schutz gesunden Gewebes bei einer Strahlentherapie eingesetzt werden.

Die nachfolgenden Beispiele sollen in Verbindung mit den Abbildungen die Erfindung näher erläutern .

Legende zu den Abbildungen:
- Abbildung 1:: Dififraktometrieanalyse des Komplexes A
- Abbildung 2:: UV/Vis - Spektrum des Komplexes A
- Abbildung 3:: IR - Spektrum des Komplexes A
- Abbildung 4 :: EPR - Spektrum des Komplexes A
- Abbildung 5 :: UV/Vis - Spektrum des Komplexes B
- Abbildung 6 :: IR - Spektrum des Komplexes B
- Abbildung 7 :: EPR - Spektrum des Komplexes B
- Abbildung 8:: UV/Vis - Spektrum des Komplexes C
- Abbildung 9 :: IR - Spektrum des Komplexes C
- Abbildung 10 :: EPR - Spektrum des Komplexes C
- Abbildung 11 :: DNA - Fragmentierung durch Komplex A auf BJAB mock
- Abbildung 12 :: DNA - Fragmentierung in primären Lymphoblasten im Vergleich zwischen Komplex A und anderen Zytostatika
- Abbildung 13 :: Änderung des mitochondrialen Membranpotenzials in BJAB - Zellen nach Behandlung mit Komplex A
- Abbildung 14 :: Synergieeffekt von Komplex A mit Cytarabin in Lymphomzellen
- Abbildung 15 :: Wirkung von Komplex A auf humane Lymphomzellen (BJAB) in der Maus bei peroraler Applikation
- Abbildung 16 :: EPR - Spektren eines Yokerkarzinosarkoms bei Behandlung mit Komplex A
- Abbildung 17 :: Enfluss von Komplex A und Komplex C auf den Zustand des NADH und die Calciumhomöostase des Timozyts des Erlich- Aszyteskarzinoms der Ratte
- Abbildung 18 :: Einfluss von Komplex A und Komplex C auf die Wirkung ionisierender Strahlung am Beispiel der Milz von Mäusen
- Abbildung 19 :: Antioxidanz - Eigenschaften von Komplex A und Komplex C
- Abbildung 20 :: Induktion von Cyclosporin A - Sensorporen durch Komplex A im Vergleich mit Sarcolysin
- Abbildung 21 :: Wirkung von Komplex A auf Zellkulturen des humanen Kehlkopfkrebses Hep - 2 (Kernspinresonanz)
- Abbildung 22 :: Wirkung von Komplex A in vitro auf humane Mammakarzinom - Zellen (Foto 1: nach 4 min. Inkubation; Foto 4: nach 18 min. Inkubation; Foto 9: nach 43 min. Inkubation)

### Beispiele

### Beispiel 1

### Herstellung eines Kupfer-Organokomplexes (Komplex A) von 4-[Bis(2-chlorethyl)-amino]-D,L-phenylalanin-hydrochlorid (= Sarcolysinhydrochlorid)

0,0125 Mol Sarcolysinhydrochlorid werden in 50 ml eines Gemischs aus 1 Teil Methanol und 3 Teilen Chloroform gelöst (Lösung A).

0,01 Mol Kupfer(II)acetat werden ebenfalls in 50 ml des obigen Lösungsmittelgemischs gelöst (Lösung B).

Lösung B wird unter ständigem Rühren und Erhitzen auf Siedetemperatur zur Lösung A gegeben. Das Gemisch wird weitere 30 Minuten unter Rühren auf Siedetemperatur gehalten und dann auf Raumtemperatur abgekühlt. Der pH-Wert der Mischung beträgt 2 bis 3.

Innerhalb einiger Stunden fällt ein feinkristalliner, grüner Niederschlag aus, der durch Filtration mittels einer Glasfritte abgetrennt, mehrfach mit dem obigen Lösungsmittelgemisch gewaschen und bei 65 bis 70°C an der Luft getrocknet wird.

Der entstandene Kupferkomplex (Komplex A) hat einen Schmelzpunkt von 147°C. Die Elementaranalyse ergibt folgende Zusammensetzung:Cu: 15,0%, C: 37,3%, H: 4,43%, Cl: 24,4%, N: 6,74%, O: 10,3%

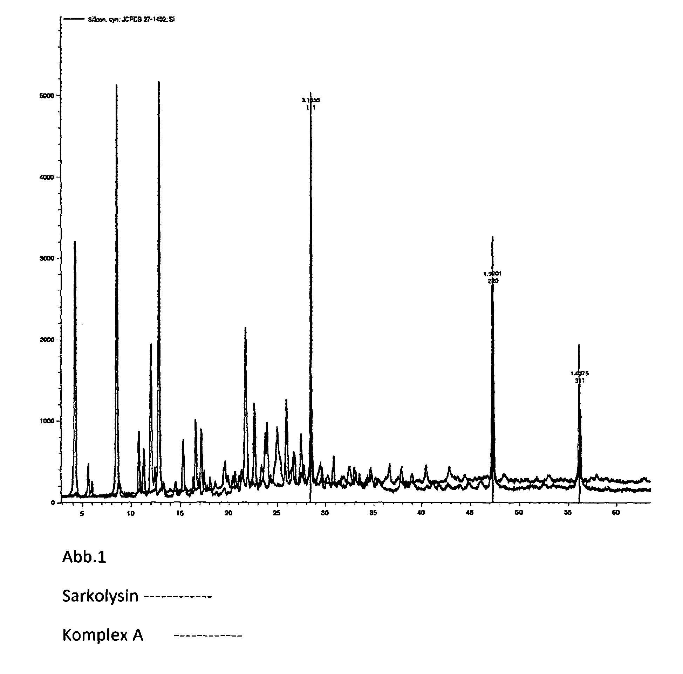

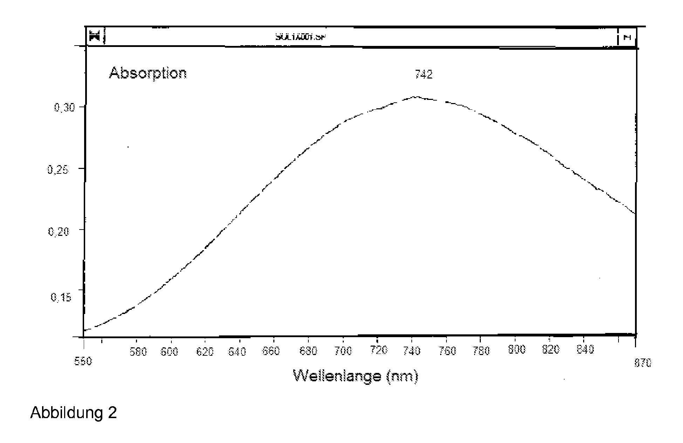

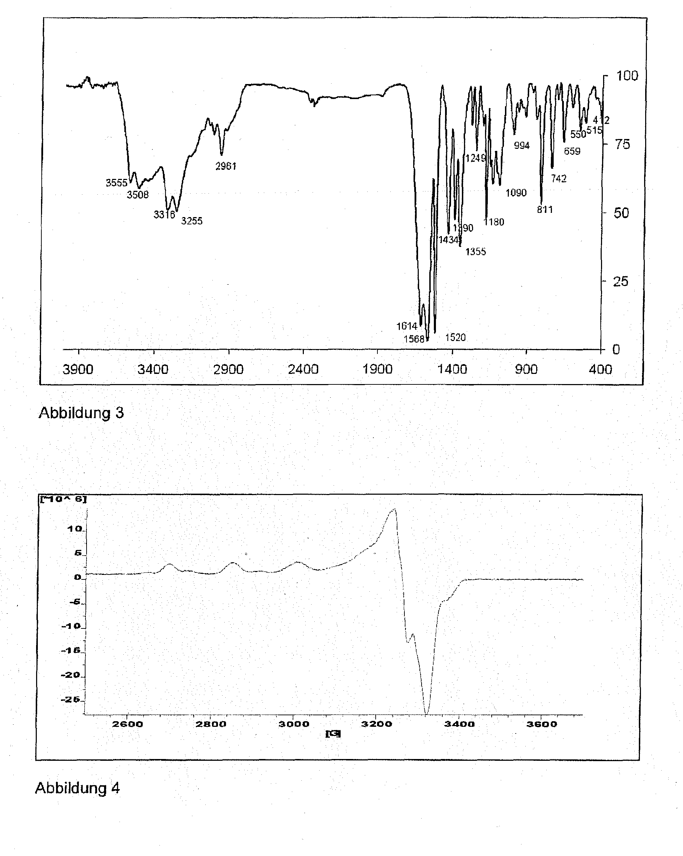

Weitere physikalische Eigenschaften des Komplexes A sind in den Abbildungen 1 bis 4 dargestellt.

Der Komplex A ist in Wasser und den meisten organischen Lösungsmitteln unlöslich. Er löst sich in Dimethylsulfoxid (DMSO ) und lässt sich in pflanzlichen Ölen und mit Hilfe geeigneter Emulgatoren (Tween 60 und Tween 80 ) in wässrigen Medien emulgieren. Er ist bei Raumtemperatur auch unter Zutritt von Sauerstoff stabil.

### Beispiel 2

### Herstellung eines Kupfer-Organokomplexes (Komplex B) von 4-[Bis(2-chlorethyl)amino-D,L-phenylalanin (= Sarcolysin)

0,0125 Mol Sarcolysin werden in 50 ml einer Mischung von 3 Teilen Chloroform und 1 Teil Methanol gelöst ( Lösung A ).
0,01 Mol Kupfer(II)acetat werden in 50 ml des vorstehenden Lösungsmittels gelöst ( Lösung B ).
Lösung B wird unter Erhitzen und ständigem Rühren zu Lösung A gegeben. Die Mischung wird unter Rühren ca. 20 Minuten auf 50°C gehalten und dann auf Raumtemperatur abgekühlt.
Der pH-Wert der Mischung beträgt 6 bis 7.
Während einiger Stunden fällt ein feinkristalliner blauer Niederschlag aus, der mittels einer Glasfritte abfiltriert, mehrmals mit dem Lösungsmittel gewaschen und bei 65 bis 70°C an der Luft getrocknet wird.
Der entstandene Kupferkomplex (Komplex B) hat einen Schmelzpunkt von 177 °C.
Die Elementaranalyse ergibt folgende Zusammensetzung:
Cu: 9,2%, C: 45,3%, H: 5,4%, Cl: 20,6%, N: 8,1%, O: 11,6%

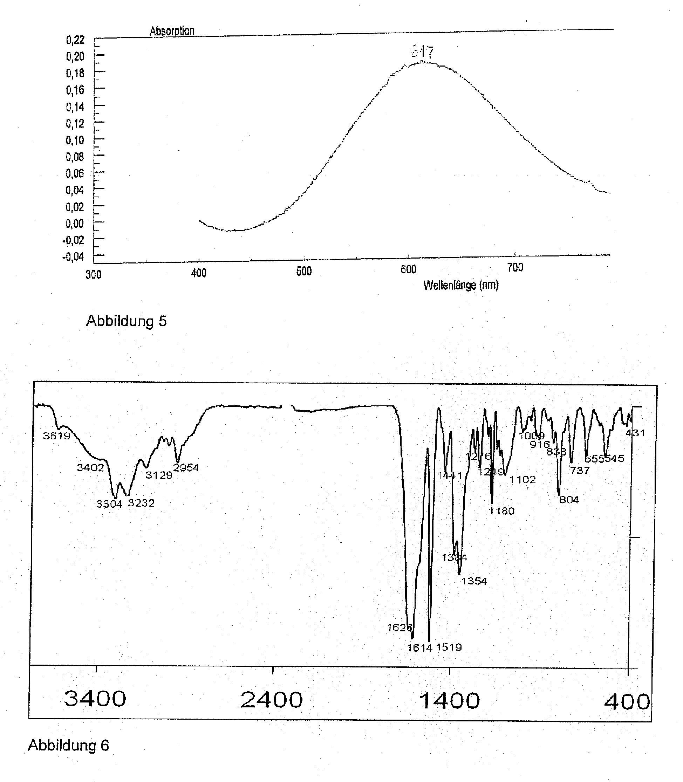

Weitere physikalische Eigenschaften des Komplexes B sind in den Abbildungen 5 bis 7 dargestellt.
Der Komplex B ist in Wasser und vielen organischen Lösungsmitteln unlöslich. Er löst sich in DMSO und lässt sich in Öl und mit Hilfe geeigneter Emulgatoren (Tween 60 und Tween 80) in wässrigen Medien dispergieren.

### Beispiel 3

### Herstellung eines Kuperferkomplexes (Komplex C) von 5-Fluoro-1-(tetrahydro-2-furyl)-uracil (= Tegafur)

### Variante 1

0,01 Mol Kupfer(II)acetat werden in 50 ml Chloroform gelöst ( Lösung A ).
0.02 Mol Tegafur in 50 ml einer Mischung aus Chloroform und Methanol im Volumenverhältnis 1:1 gelöst ( Lösung B ).
Die Lösungen A und B werden im Wasserbad zum Sieden erhitzt, unter ständigem Rühren zusammengegeben und mit Salzsäure angesäuert. Die Mischung wird in einem offenen Gefäß im Dunkeln stehen gelassen, bis die Lösungsmittel verdampft sind. Der Rückstand wird mit Chloroform von Ausgangssubstanzen frei gewaschen und die zurückbleibenden grünen Kristalle an der Luft getrocknet.
Der entstandene Kupferkomplex (Komplex C) hat einen Schmelzpunkt von 127°C.

### Variante 2

0,01 Mol Kupfer(II)acetat werden in 50 ml Chloroform/Methanol im Vol. - Verhältnis 1:1 gelöst (Lösung A).
0,02 Mol Tegafur werden in 50 ml des vorstehenden Lösungsmittels gelöst (Lösung B).
Die beiden Lösungen werden zum Sieden erhitzt und Lösung A unter ständigem Rühren in Lösung B gegeben. Die Mischung wird weitere 30. Min bei Siedetemperatur gehalten, wobei der pH-Wert durch Zudosierung von konzentrierter HCl auf 1 gehalten wird. Die reagierte Mischung wird bei Raumtemperatur eingedampft und der trockene Rückstand in einer Glasfritte 3 bis 4 mal mit Methanol von -18°C gewaschen. Die Waschflüssigkeit enthält den erfindungsgemäßen Komplex C, der nach dem Verdampfen des Methanols als Rückstand verbleibt.

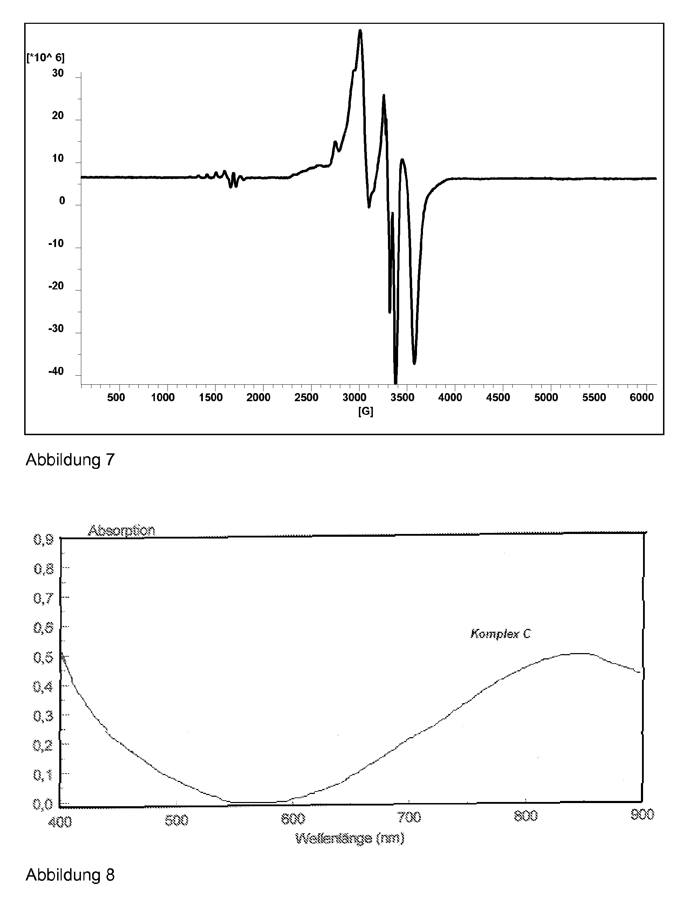

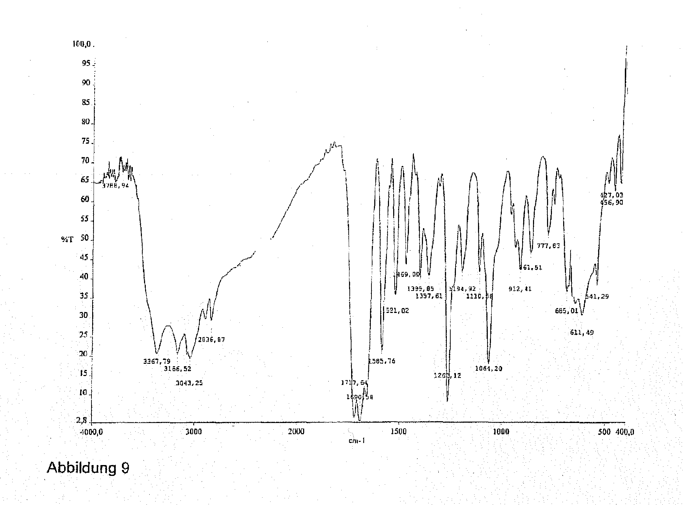

Weitere physikalische Eigenschaften des Komplexes C sind in den Abbildungen 8 bis 10 dargestellt.

Der Komplex C löst sich gut in Wasser, physiologischer Kochsalzlösung, Methanol, Ethanol, DMSO und Tween 80. Er ist unlöslich in Ether und Chloroform. Er ist bei Raumtemperatur an trockener Luft stabil.

### Beispiel 4

### Einfluss von Komplex A auf die Apoptoseinduktion

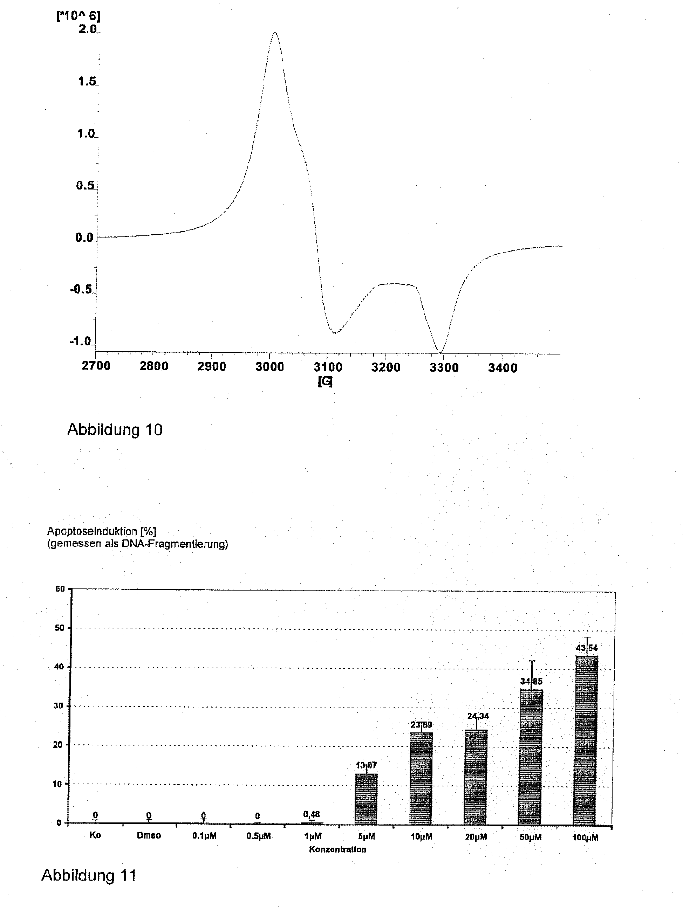

BJAB-Zellen (Lymphom-Zelllinie) in einer Konzentration von 1x 10⁵/ml wurden mit ansteigenden Konzentrationen von Komplex A, gelöst in DMSO, behandelt und 72 Stunden bei 37°C und 5% CO2 inkubiert. Nach Propidiumiodid-Färbung wurden die DNA-Fragmente mittels Durchflusszytometrie (FACS-Analyse) detektiert. K0 (unbehandelte Zellsuspension) und DMSO wurden bei äquivalenter Behandlung mitgeführt. Das Ergebnis ist in Abbildung 11 dargestellt. Es zeigt sich eine konzentrationsabhängige Apoptoseinduktion durch Komplex A, die bei 0,1 mMol Komplex A den Wert von 43,5% der Zellen erreicht.

### Beispiel 5

### Wirkung von Komplex A in primären Lymphoblasten ex vivo.

Nach der Gewinnung von ALL-Patienten wurden die Lymphoblasten zunächst isoliert und dann sowohl mit handelsüblichen Zytostatika als auch mit dem Komplex A behandelt. Dabei wurden die Konzentrationen so gewählt, dass sie sich stets im jeweiligen Bereich der LC50 bei Verwendung der Leukämie-Zelllinie NALM-6 befanden. Danach wurden die Zellen für 60 Stunden bei 37°C und 5% CO2 inkubiert, anschließend mit Propidiumiodid gefärbt und durchflusszytometrisch im FACS quantifiziert. Die Ergebnisse sind in Abbildung 12 dargestellt. Dabei zeigt sich eindrucksvoll die Apoptoseinduktion in gegen Daunorubicin (Dauno) und Doxorubicin (Doxo) resistenten Leukämiezellen. Komplex A induziert mit etwa 26% deutlich mehr Apoptose in Lymphoblasten als die herkömmlichen Zytostatika Cytarabin (ARA-C), Cladribin (Cla), Etoposid (Etop), Fludarabin (Flu) und Idarubicin (Ida).

### Beispiel 6

### Nachweis einer mitochondrialen Vermittlung der Apoptosesignalkaskade durch Komplex A

Nach Behandlung der BJAB-Zellen mit verschiedenen Konzentrationen des Komplexes A unter Mitführung einer Nullkontrolle und einer Lösungsmittelkontrolle werden die Zellen 48 stunden bei einer Temperatur von 37°C und 5% CO2 inkubiert. Die Auswertung der Versuche durch Anfärben mit dem mitochodrienspezifischen Farbstoff JC-1 und durchflusszytometrische Detektion des Farbumschlags zeigt Abbildung 13. Es zeigt sich in mehr als 30% der Zellen eine durch den Komplex A induzierte konzentrationsabhängige Änderung des mitochondrialen Membranpotentials.

### Beispiel 7

### Kombinierte Gabe von Komplex A und dem Cytostatikum Cytarabin

Das Kombinationspräparat zeigt *in vitro* ausgeprägte Synergieeffekte mit dem herkömmlichen Cytostatikum Cytarabin in Lymphomzellen (BJAB). Die Inkubation erfolgte über 48 Stunden bei 37°C und 5% CO2 unter Mitführung von Null- und Lösungsmittelkontrollen. Die DNA-Fragmentierung wird nach Färbung der Zellen mit Propidiumiodid durchflusszytometrisch bestimmt. Abbildung 14 zeigt die Mittelwerte von Dreifachmessungen. Man erkennt einen deutlichen Synergieeffekt von Komplex A mit Cytarabin von + 129% der Apoptoseinduktion.

### Beispiel 8

### Hemmung des Tumorwachstums in vivo an der Maus unter Verwendung von Komplex A

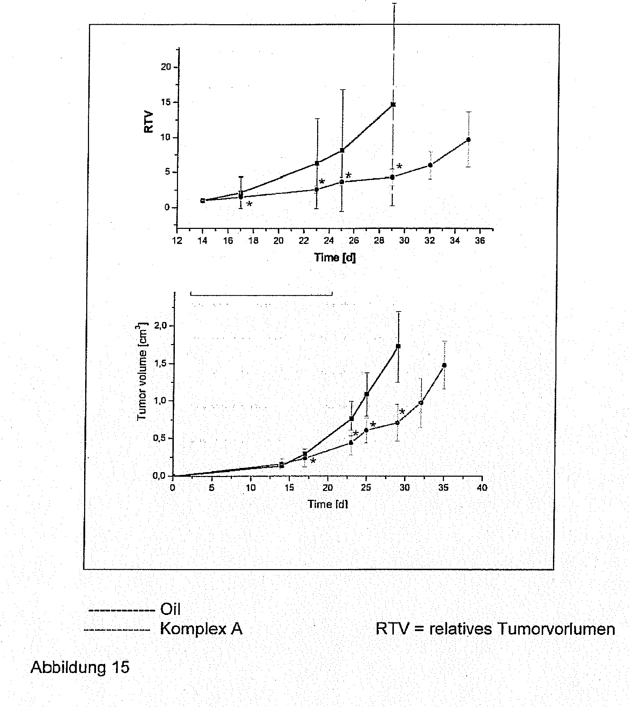

Sechs SCID-Mäusen wurden humanen Lymphomzellen (BJAB) implantiert. Als ein Tumor mit einem Durchmesser von 5 mm entstanden war, wurden die Mäuse mit Komplex A suspendiert in Olivenöl in einer Dosis von 10 mg/kg peroral behandelt. Die Kontrollgruppe bestand aus 10 nur mit Olivenöl behandelten Tieren. Nach der Gabe von Komplex A wurde eine signifikante Inhibierung des Tumorswachstums beobachtet. Das Ergebnis ist in Abbildung 15 dargestellt.
Dabei bedeutet * die Signifikanz (p≤0,05) im Man-Whitney- U-Test.

### Beispiel 9

### Wirkung von Komplex A auf das Melanom B 16 der Maus

20 Mäusen der Linie C 57BI wurde durch subkutane Injektion in den rechten Oberschenkel das Melanom B 16 implantiert. Die Tiere wurden auf 4 Gruppen von je fünf Mäusen aufgeteilt. Gruppe I erhielt Komplex A in einer Dosierung von 7 mg/kg, gelöst in DMSO, das in einer 4%igen Suspension in physiologischer Kochsalzlösung intraabdominal appliziert wurde. Die Applikation erfolgte jeweils am 3., 5., 7. und 9. Tag nach der Transplantation des Tumors.
Gruppe II wurde wie Gruppe I behandelt, jedoch mit einer Dosierung von 5 mg/kg. Gruppe III wurde wie Gruppe I behandelt, erhielt jedoch 5 mg/kg Melphalan-Hydrochlorid anstelle von Komplex A.
Gruppe IV wurde nicht behandelt.
Tabelle 1 zeigt für die 4 Gruppen jeweils das durchschnittliche Gewicht und Volumen des Tumors, die Inhibierung des Tumorwachstums, den durchschnittlichen Mitose-und Apoptoseindex und den Mitoseindex des Knochenmarks.
Aus der Tabelle 1 geht deutlich die überlegene Antitumorwirkung des Komplexes A hervor und eine deutlich geringere Hemmung der Produktion von Knochenmark.

**Tabelle 1**

| Substanz | Durchschnittliches Gewicht und Volumen (cm³) des Tumors | Inhibierung des Tumorwachstums % | Mitoseindex der Tumorzellen MI ‰ | Apoptoseindex Al ‰ | Knochenmark-index MI KM ‰ |
|---|---|---|---|---|---|
| I. Gruppe Komplex A | 136,8 mg | Masseprozent 89,5 | 1,3 | 4,25 | 3,575 |
| | 0,029 cm³ | Volumenprozent 98,67 | | | |
| II. Gruppe Komplex A | 202,25 mg | Masseprozent 84,44 | 2,175 | 3,68 | 3,975 |
| | 0,436 cm³ | Volumenprozent 80,00 | | | |
| III. Gruppe Positiv Kontrolle-Sarcolysin | 482,6 mg | Masseprozent 62,87 | 2,43 | 3,07 | 1,88 |
| | 1,4 cm³ | Volumenprozent 35,78 | | | |
| IV. Gruppe Kontrolle | 1300 mg | | 4,84 | 3,84 | 4,81 |
| | 2,18 cm³ | | | | |

### Beispiel 10

### Vergleich der Wirkung von Komplex A auf das Melanom B 16 der Maus bei peroraler Applikation und intraabdominale Injektion

23 Mäusen der Linie C 57B1 wurde durch subkutane Injektion in den rechten Oberschenkel das Melanom B 16 implantiert. Die Tiere wurden auf fünf Gruppen von je vier bis fünf Mäusen aufgeteilt.
Gruppe I erhielt Komplex A in einer Dosierung von 7 mg/kg, als Suspension in Olivenöl, die peroral jeweils am 3., 5., 7. und 9. Tag nach der Transplantation des Tumors verabreicht wurde.
Gruppe II wurde wie Gruppe I behandelt, jedoch mit einer Dosierung von 10 mg/kg und Applikation jeweils am 3., 4., 5., 6., 7., 8., 9. und 10. Tag.
Gruppe III wurde wie Gruppe II behandelt jedoch mit einer Dosierung von 15 mg/kg.
Gruppe IV erhielt den Komplex A jeweils am 3., 5., 7. und 9. Tag nach der Transplantation des Tumors in einer Dosierung von 7 mg/kg, gelöst in DMSO, wobei die Lösung als 4%ge Suspension in physiologischer Kochsalzlösung durch Injektion appliziert wurde.
Gruppe V erhielt keine Behandlung.
Die Tiere wurden am 16. Tag des Experiments getötet und untersucht.
Die Versuchsergebnisse sind in Tabelle 2 zusammengestellt. Es zeigt sich, dass die Antitumorwirkung unabhängig von der Applikationsmethode ist. In der Gruppe 3 ist der Tumor sogar vollständig verschwunden.

**Tabelle 2**

| Substanz | Durchschnittliches Gewicht (mg) und Volumen (cm³) des Tumors | Hemmung % |
|---|---|---|
| I.Gruppe Komplex A (peroral) | 0,96±0,14 | Masseprozent 62,0 |
| | p>0.05 | |
| | 1,39 cm³ | Volumenprozent 84,1 |
| II. Gruppe Komplex A (peroral) | 0,68±0,34 | Masseprozent 73,10 |
| | 0,1>p>0.05 | |
| | 1,55 cm³ | Volumenprozent 76,0 |
| III. Gruppe Komplex A (peroral) | 0 | Masseprozent 100 |
| | p<0.05 | |
| | 0 | Volumenprozent 100 |
| IV. Gruppe Komplex A (Injektion) | 1,025±0,39 | Masseprozent 68,0 |
| | p>0.05 | |
| | 1,5 cm³ | Volumenprozent 76,7 |
| V. Gruppe Kontrolle | 2,525±0,34 | - |
| | 6,46 cm³ | |

### Beispiel 11

### Wirkung von Komplex A auf den transplantierten Adenokarzinomtumor des Dickdarms (AKATOL) bei inraabdominaler Injektion.

15 Mäusen der BALB - Linie wurde eine Suspension der Tumorzellen in den Oberschenkel injiziert. Die Tiere erhielten das Komplex A- Präparat in einer Dosis 5 mg/kg jeweils am 4., 6., 8. und 11. Tag nach der Implantation.
Gruppe I erhielt das Präparat gelöst in DMSO, das in einer 4%igen Suspension in physiologischer Kochsalzlösung dispergiert wurde.
Gruppe II erhielt das Präparat in Tween 60.
Gruppe III wurde nicht behandelt.
Die Tiere wurden am 21. Tag des Experiments getötet und untersucht.
Tabelle 3 zeigt die Ergebnisse. Die Verabreichung in Tween 60 zeigt eine deutliche bessere Wirkung. Offenbar bewirkt das inerte Detergenz eine bessere Verteilung des Komplexes A im Organismus.

**Tabelle 3**

| Gruppe | Durchschnittliches Volumen des Tumors (cm³) | Durchschnittliches Gewicht (mg) | Hemmung % | |
|---|---|---|---|---|
| | | | Masseprozent | Volumenprozent |
| I | 1,315 | 1,25 | 60,4 | 87,1 |
| II | 0,217 | 0,25 | 95,85 | 97,8 |
| III | 10,164 | 6,02 | | |

### Beispiel 12

### Wirkung des Komplexes A auf des transplantierten Adenokarzinomtumor des Dickdarms (AKATOL) bei peroraler Applikation

Mäusen der BALB-Linie wurden die Tumorzellen durch Injektion im Oberschenkel transplantiert. 3 der 4 Gruppen erhielten jeweils am 3., 4., 5., 6., 7., 8., 89. und 10. Tag nach der Tumorimplantation peroral die jeweilige Zubereitung:
Gruppe I: 10 mg/kg Komplex A suspendiert in Olivenöl.
Gruppe II: 5 mg/kg Komplex A suspendiert in Olivenöl.
Gruppe III: 10 mg/kg Sarcolysin suspendiert in Olivenöl.
Gruppe IV: erhielt keine Behandlung.
Die Tiere wurden am 21. Tag des Experiments getötet und untersucht. Die Ergebnisse sind in Tabelle 4 zusammengestellt. Auch bei peroraler Applikation zeigt Komplex A eine gegenüber Sarcolysin (Melphalan) signifikant erhöhte Wirksamkeit.

**Tabelle 4**

| Substanz | Dosis mg/kg | Durchschnittliches Gewicht des Tumors (g) | Hemmung % |
|---|---|---|---|
| I. Gruppe | | | |
| Komplex A | 10 | 0,71 | 81,5 |
| II. Gruppe | | | |
| Komplex A | 5 | 1,04 | 68 |
| III. Gruppe | | | |
| Positiv | | | |
| Kontrolle | 10 | 1,85 | 43 |
| Sarcolysin | | | |
| IV. Gruppe | | | |
| Kontrolle | | 3,25 | |

### Beispiel 13

### Wirkung des Komplexes A auf das Ascites Ehrlich - Adenokarzinom

Weißen rasselosen Mäusen wurde der Tumor intraabdominal implantiert.
Gruppe I erhielt Komplex A als Suspension in Olivenöl peroral in einer Dosierung von 10 mg/kg jeweils am 3., 4., 5., 6., 7., 8., 9. und 10. Tag nach der Tumorimplantation. Gruppe II erhielt die gleiche Behandlung, jedoch mit Sarcolysin anstelle von Komplex A.
Gruppe III erhielt keine Behandlung.
Die Tiere wurden am 11. Tag nach der Tumorimplantation getötet und analysiert. Die Ergebnisse sind in Tabelle 5 zusammengestellt.
Komplex A inhibiert die Proliferation der Tumorzellen deutlich besser als Sarcolysin und supprimiert nicht die Produktion der roten Knochenmarkszellen.

**Tabelle 5**

| Substanz | Durchschnittliche Anzahl der Tumorzellen (10⁶) | Hemmung % | Durchschnittlicher MI des Knochenmarks, ‰ | Durchschnittlicher MI der Tumorzellen ‰, |
|---|---|---|---|---|
| I. Gruppe Komplex A | 40 | 75,3 | 0,95±0,47 | 1,0±0,7 |
| II. Gruppe Positiv Kontrolle - Sarcolysin | 77 | 47,4 | 0,52±0,26 | 2,0±1,87 |
| III. Gruppe Kontrolle | 162,4 | 1,3 | 1,0±0,31 | 3,2±0,44 |

### Beispiel 14

### Einfluss des Komplexes A auf die Bildung von Nitrosylkomplexen des Häm - Eisens am Beispiel Yokerkarzinosarkom

In malignen Zellen wird der Sauerstoff -Stoffwechsel, dadurch beeinträchtigt, dass die erhöhte NO - Produktion dieser Zellen zu einer erhöhten Bindung von NO an das Häm - Eisen führt, das dadurch in seiner normalen Funktion gestört wird. Die Bildung solcher NO-Bindungen manifestiert sich im EPR-Spektrum in einem charakteristischen Triplett, das einem Singulett aufgesetzt ist.

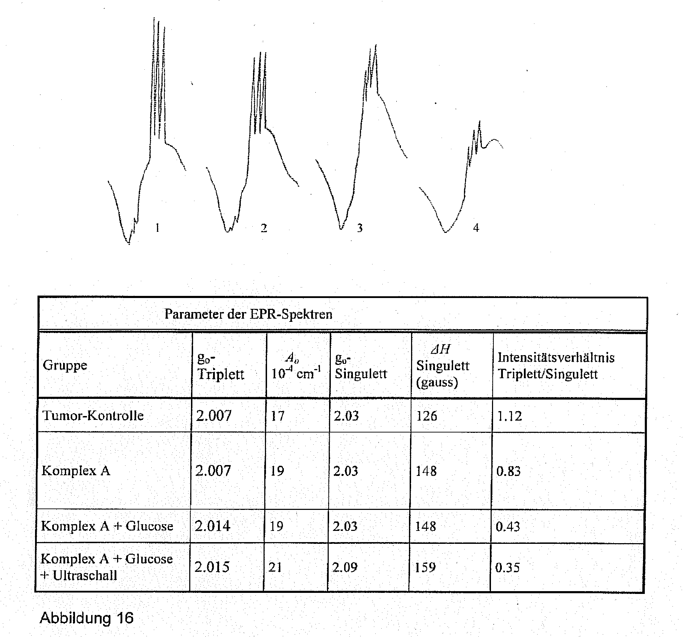

In einem Versuch mit Ratten wurde die Wirkung von Komplex A auf die Konzentration der Fe-NO-Bindungen untersucht. Den Ratten wurde das Yokerkarnozisarkom transplantiert. 17 Tage nach der Transplantation wurde den Tieren intraabdominal Komplex A in einer Dosierung von 30 mg/kg suspendiert in Liposomen aus Eierlezithin appliziert. Ein Teil der Tiere erhielt gleichzeitig Glukose oder Glukose plus Ultraschall. Die Ergebnisse sind in Abbildung 16 zusammengestellt. Es zeigt sich, dass Komplex A insbesondere in Zusammenwirken mit Hyperglykämie und insbesondere Hyperglykämie plus Ultraschall zu einer signifikanten Verringerung der Konzentration der Fe-NO-Bindungen führt.

### Beispiel 15

### Wirkung des Komplexes A auf das NADH und die Calciumhomöostase von Ratten und den Thymozyt und Thymus des Ehrlich-Ascyteskarzinoms

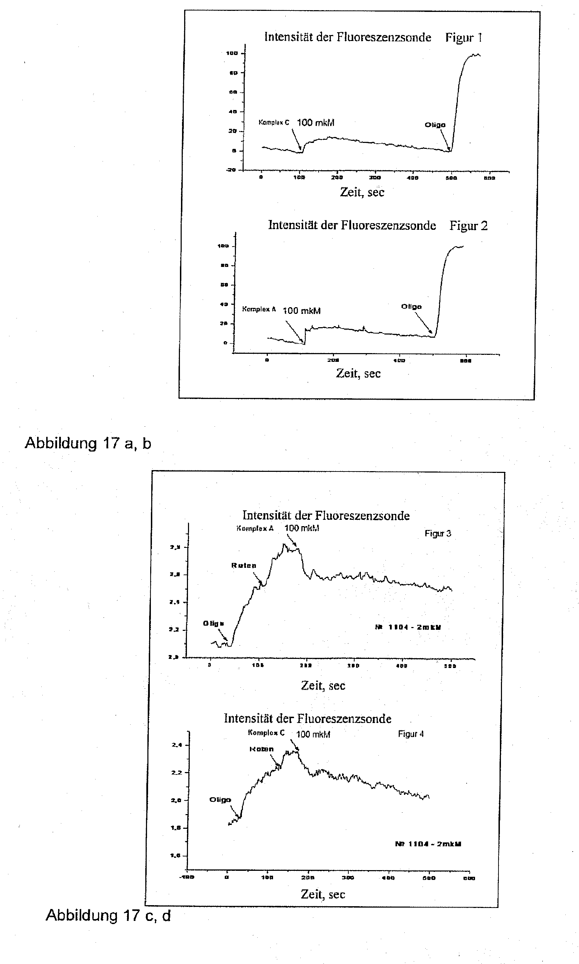

Das Ehrlich-Ascyteskarzinom (EAK) wurde von weißen männlichen Mäusen des Stammes MNRI gewonnen, das Thymozyt aus dem Thymus von Ratten der Wistar-Linie. Die Vitalität der Zellen wurde mit Vitalfärbung mittels Trypanblau und Experimenten geprüft und in allen Experimenten auf nicht weniger als 95% festgestellt. Der Zustand des NADH in den EAK-Zellen wurde mittels deren Fluoreszenzintensität bewertet. Die Messung der intrazellulären Calciumkonzentration wurde nach der Standartmethodik Fluoreszenz mit Chlortetrazyklin bestimmt. Das Membranpotenzial wurde mit Hilfe des Fluoreszenzfarbstoffs Nr. 1104 gemessen. Das NADH ist eine Quelle von Elektronen im oxydativen Phosphorylierungsprozess. Es zeigte sich, dass die Komplexe A und C auf den Zustand des NADH und die Calciumkonzentration einwirken. Sie vergrößern die Menge des endogenen NADH auf mehr als 20% und vermindern die Reaktion auf Olygomyzin (Abb. 17a, 17b). Diese Effekte wirken auf die Inhibierung der NAD-abhängigen Dehydrogenase hin. Im Ergebnis wird eine Hemmung der Atmungskette in den Tumorzellen bewirkt.

Des Weiteren konnte gezeigt werden, dass die Komplexe A und C abhängig von der Dosis die Konzentration des zytosolen Calciums in den EAK-Zellen und den Thymozyten steigern. Die Konzentration des intrazellulären Calcium ist der Auslöser für mehrere intrazelluläre Prozesse (Abb. 17c, 17d).

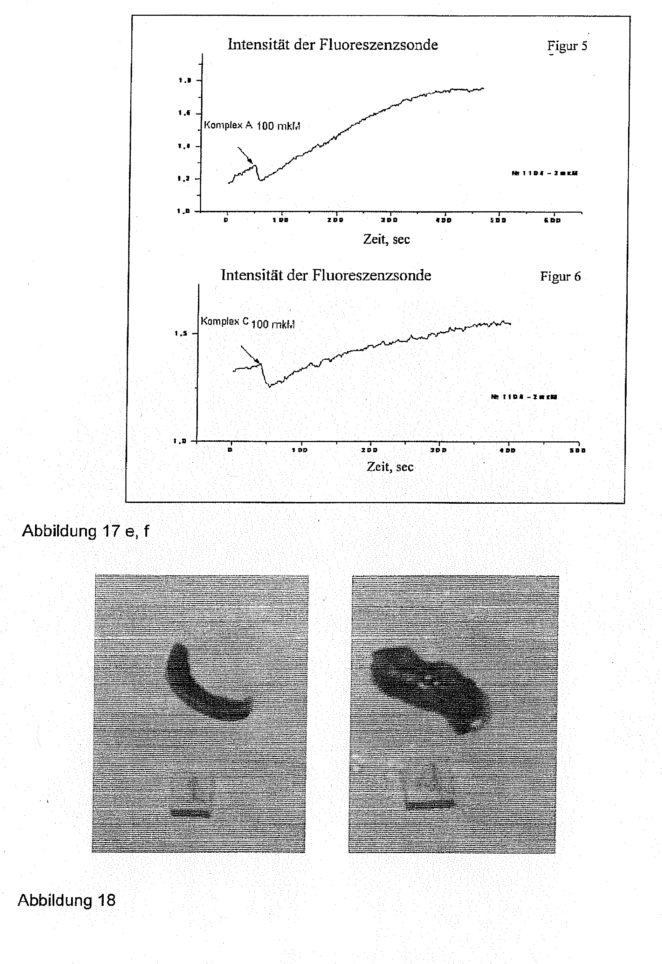

Die Komplexe A und C beeinflussen darüber hinaus das Membranpotenzial der Thymozyte. Es wurde gefunden, dass die Hyperpolarisation der Zellmembranen (Abb. 17e, 17f) eine Öffnung der Calciumkanäle und Depolarisation der Mitochondrialmembranen auslöst.

### Beispiel 16

### Strahlenschutzwirkung von Komplex A und Komplex C

Weiße rasselose Mäuse wurden mit der subletalen Dosis von 6 Gray bestrahlt.

Ein Teil der Mäuse wurde zwei Stunden vor der Bestrahlung und drei Tage nach der Bestrahlung mit Komplex A und Komplex C in einer Dosis von je 5 mg/kg behandelt. Die Tiere wurden am 9. Tag nach der Bestrahlung getötet und die Anzahl der blutbildenden Zellen in der Milz bestimmt. Bei Tieren, die nicht behandelt wurden, waren in der Milz keine blutbildenden Zellen nachzuweisen. Die behandelten Tiere wiesen die normale Anzahl von ca. 40 Kolonien blutbildenden Zellen auf.

In Abbildung 18 ist vergleichend die Milz einer unbehandelten und einer behandelten Maus dargestellt.

### Beispiel 17

### Antioxidanzeigenschaften von Komplex A und Komplex B

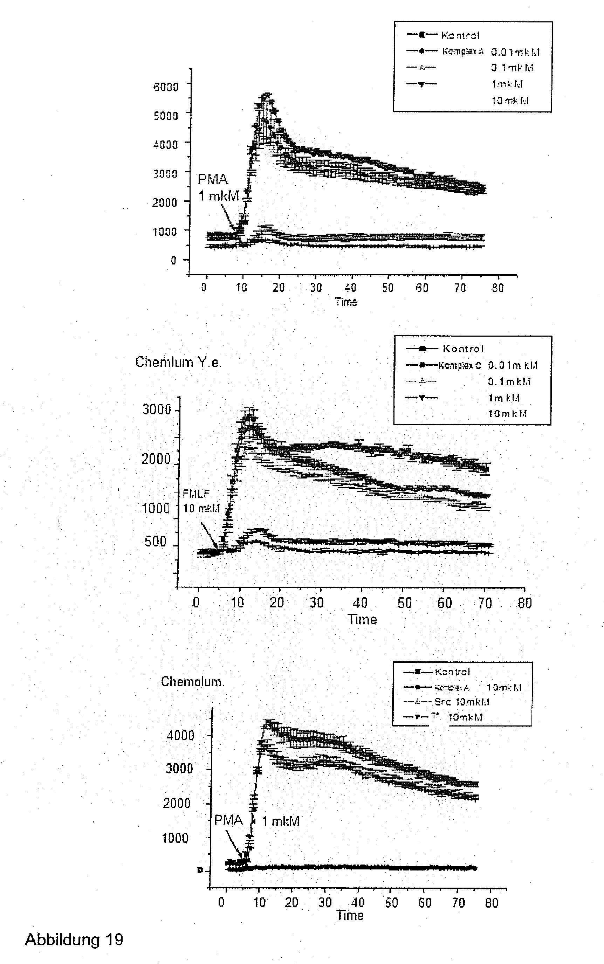

In einem Milieu, das einen Überschuss an reaktiven Sauerstoffspezies (ROS=reactive oxygen species) hat, zeigen die Komplexe A und C eine ausgeprägte Wirkung als Antioxidationsmittel So wurde in Neutrophilen, die aus männlichen Mäusen der NMRI-Linie erhalten wurden, die ROS-Bildung durch zwei Aktivatoren ausgelöst: phorbol-12-myristat-13-acetat (PMA) und N-formyl-methionyl-leicylphenylalanin (FMLF). Abbildungen 19a und b zeigen, dass durch die Zugabe von Komplex A und Komplex C abhängig von der Dosierung die Bildung von ROS in den Neutrophilen vermindert bzw. ganz unterdrückt wird. Abbildung 19c zeigt dass dieser Effekt mit Sarcolysin und Tegafur nicht annähernd erreicht wird.

### Beispiel 18

### Wirkung der Komplexe A und C im Vergleich zu Sarcolysin auf die Durchlässigkeit der Mitochondrienmembran in Leberzellen von Ratten

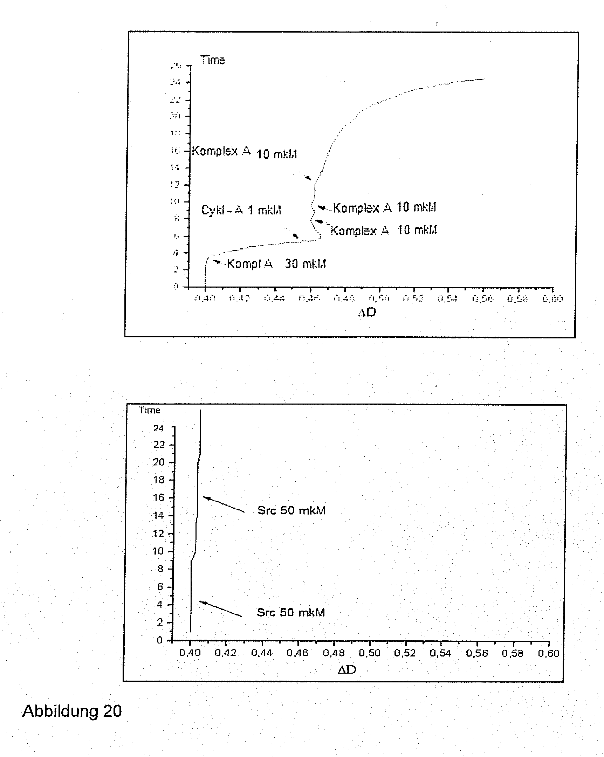

Als Indikator für die Durchlässigkeit wird die optische Dichte der Zellsuspension gemessen. Es zeigt sich, dass die Komplexe A und C einen starken Einfluss auf die Durchlässigkeit haben, der durch Zugabe von Cyklosporin-A gestoppt bei weiterer Zugabe der Komplexe aber wieder hergestellt wird (Abb. 20a). Abbildung 20b zeigt, dass Sarcolysin diesen Einfluss nicht hat.

Es wurde festgestellt, dass die Komplexe A und C einen hohen Einfluss auf die Durchlässigkeit der Mitochondrienmembranen haben. Sie öffnen die Cyklosporin-A-Sensorporen bis zu hoher Durchlässigkeit (bis 1500 Dalton). Sie reduzieren irreversibel das Membranpotenzial. Sie führen zur osmotischen Schwellung der Mitochondrienmatrix. Sie tragen zur Zerstörung der Mitochondrienmembran bei. Der Hauptfaktor dieser Wirkung ist offenbar das Kupfer als Zentralatom des Komplexes. Es verändert nicht nur die Eigenschaften des Sarcolysin und Tegafur sondern entfaltet offenbar auch selbst biologische Wirkungen.

### Beispiel 19

### Wirkung von Komplex A auf Tumorzellen des menschlichen Kehlkopfkrebses Hep-2

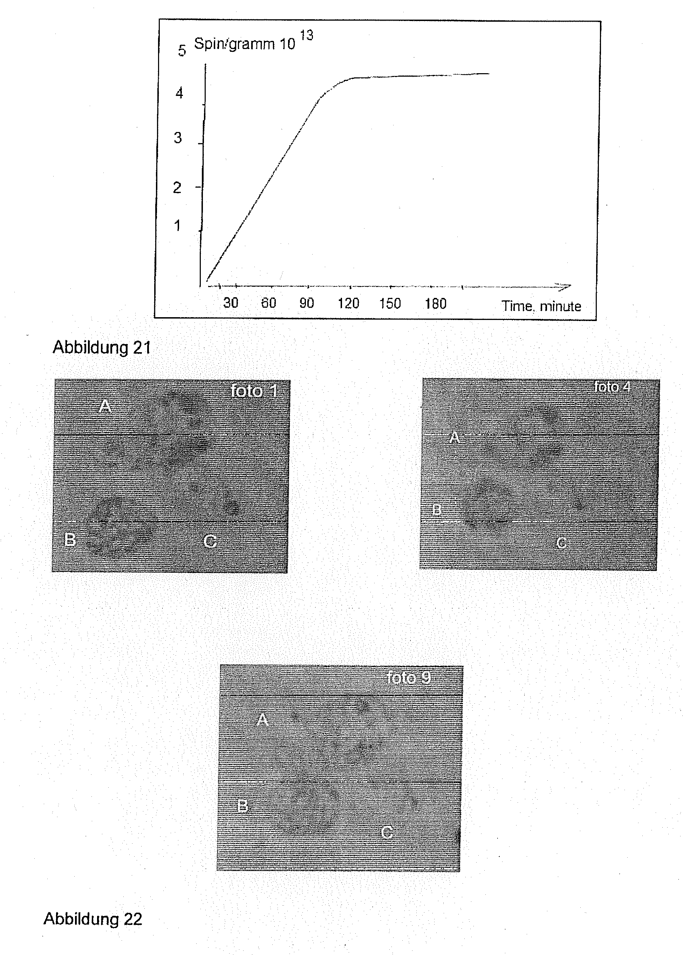

Komplex A gelöst in Tween-80 wurde mit Liposomen aus Lecithin vermischt. Diese Mischung wurde einer Kultur von Hep-2 Zellen zugesetzt und die EPR-Spektren über den Zeitablauf registriert. Als Vergleich wurde die Kultur ohne das Komplex A Präparat gemessen. Aus EPR-Spektren lässt sich der Kupfergehalt in den Tumorzellen quantitativ bestimmen. Es stellte sich heraus, dass die Hep-2 Zellen Kupfer in einer Menge von 4,5x10¹³ Spin pro Gramm aufnehmen. Das entspricht einer Aufnahme von 10 Kupferhaltigen Molekülen in eine Zelle. Dieser Sättigungswert der Zellen wird innerhalb von 1,5 bis 2 Stunden erreicht (Abb. 21). Zusätzlich wurde die Zellesuspension durch Filtration gereinigt und das Filtrat, das die Zellbestandteile enthielt, mit einer Zentrifuge fraktioniert. Dabei zeigte sich, dass sich der Kupferkomplex fast vollständig im Kernchromatin anreichert, wie durch Messung des EPR-Signals des Kupfers festgestellt wurde.

### Beispiel 20

### Wirkung von Komplex A in vitro auf Tumorzellen der menschlichen Milchdrüse

Einer Patientin mit einem Mammakarzinom, das bereits Zerfallserscheinungen zeigte, wurde postoperationelles Material entnommen, aus dem Tumorzellen abgetrennt wurden. Einer Suspension dieser Tumorzellen wurde der Farbstoff Trypanblau und Komplex A zugesetzt. Während des Versuchsverlaufs wurden drei nahe bei einander liegende Zellen (A, B und C) fotographisch dokumentiert (Abb. 22a, 22b und 22c). Nach 18 Minuten Inkubation erkennt man eine Fältelung der Membran von Zelle A und eine Invagination der Membran der Zelle B. Nach 28 Minuten Inkubation verfärben sich Zellfragmente trypanblau, ein Zeichen dafür, dass die Apoptose eingesetzt hat. Nach 43 Minuten der Inkubation fallen Membranteile samt Inhalt von den Zellen ab.

## Patentansprüche

1. Kupfer-Organokomplex, erhalten durch Umsetzung eines Kupfer(II)acylats bei saurem bis neutralem pH-Wert in einem Chlorofom/Methanolgemisch mit einer organischen Verbindung ausgewählt aus 4-[Bis(2-chlorethyl)-amino]-D,L-phenylalanin (Sarcolysin), dessen Hydrochlorid, N-(2-Furanidil)-5-fluoruracil (Tegafur) und Aminocarbonylaziridin (2-Carbamoylaziridin - Leacadin).

2. Kupfer-Organokomplex nach Anspruch 1, **dadurch gekennzeichnet, dass** als Kupfer(II)acylat Kupfer(II)acetat oder Kupfer(II)propionat eingesetzt wird.

3. Kupfer-Organokomplex nach Anspruch 1 oder 2, **dadurch gekennzeichnet, dass** es ein Kupfer-Sarcolysin-Hydrochlorid-Komplex mit einem Schmelzpunkt von 147°C und der Zusammensetzung: Cu: 15%, C: 37,3%, Cl: 24,4%, N: 6,74% und O: 10,3% ist, hergestellt durch Umsetzung von Sarcolysin-Hydrochlorid und Kupfer(II)acetat bei einem pH von ca. 2 bis ca. 3.

4. Kupfer-Organokomplex nach Anspruch 1 oder 2, **dadurch gekennzeichnet, dass** es ein Kupfer-Sarcolysin-Komplex mit einem Schmelzpunkt von 177°C und der Zusammensetzung: Cu: 9,2%, C: 45,3%, Cl: 20,6%, N: 8,1% und O: 11,6% ist, hergestellt durch Umsetzung von Sarcolysin und Kupfer(II)acetat bei einem pH von ca. 6 bis ca. 7.

5. Kupfer-Organokomplex nach Anspruch 1 oder 2, **dadurch gekennzeichnet, dass** es ein Kupfer-Tegafur-Komplex mit einem Schmelzpunkt von 127°C ist , hergestellt durch Umsetzung von Tegafur und Kupfer(II)acetat bei einem pH von ca. 1.

6. Pharmazeutische Zusammensetzung umfassend mindestens einen Kupfer-Organokomplex gemäß einem der Ansprüche 1 bis 5 sowie gegebenenfalls pharmazeutische Hilfs- und/oder Trägerstoffe.

7. Kombinationspräparat umfassend mindestens einen Kupfer-Organokomplex gemäß einem der Ansprüche 1 bis 5 und mindestens ein an sich bekanntes Zytostatikum sowie gegebenenfalls pharmazeutische Hilfs- und/oder Trägerstoffe.

8. Kupfer-Organokomplex nach einem der Ansprüche 1 bis 5 zur Behandlung von Tumorerkrankungen und/oder zum Schutz gesunden Gewebes vor ionisierender Strahlung.

9. Kupfer-Organokomplex nach Anspruch 8 zur Verwendung in der Strahlentherapie.

10. Kupfer-Organokomplex nach einem der Ansprüche 1 bis 5 zum in vivo Nachweis maligner Zellen

11. Kombinationspräparat nach Anspruch 7 zur Behandlung von Tumorerkrankungen und/oder zum Schutz gesunden Gewebes vor ionisierender Strahlung.

12. Verfahren zur Herstellung eines Kupfer-Organokomplexes, **dadurch gekennzeichnet, dass** Kupfer(II)acylat bei saurem bis neutralem pH-Wert in einem Chlorofom/Methanolgemisch mit einer organischen Verbindung ausgewählt aus 4-[Bis(2-chlorethyl)-amino]-D,L-phenylalanin ( Sarcolysin), dessen Hydrochlorid, N-(2-Furanidil)-5-fluoruracil (Tegafur) und Aminocarbonylaziridin (2-Carbamoylaziridin - Leacadin) umgesetzt wird.
